# EUROPEAN PATENT SPECIFICATION

(11) **EP 0 571 085 B1**
(45) Date of publication and mention of the grant of the patent: **31.07.1996**
(21) Application number: 93303115.5
(22) Date of filing: 21.04.1993
(51) Int. Cl.: C12N 15/54, C12N 9/12, C12P 21/00, C12N 5/10, C12Q 1/50

(54) **Stable isoforms of creatine kinase for standard markers and diagnosis**
Stabile Isoformen von Creatine-Kinase zur Verwendung als Standardmarker und für die Diagnose
Isoformes stables de créatine kinase comme marqueurs standards et pour le diagnostic

(30) Priority: 21.04.1992 US 871469
(43) Date of publication of application: 24.11.1993
(73) Proprietor: BAYLOR COLLEGE OF MEDICINE, Houston, TX 77030 (US)
(72) Inventor: Perryman, M. Benjamin, Denver,Colorado 80222 (US); Kesterson, Robert, Escondido, California 92037 (US); Friedman, David L., Houston, Texas 77054 (US); Roberts, Robert, Houston, Texas 77019 (US)
(74) Representative: Hallybone, Huw George

(56) References cited:
- EP-A- 0 423 953
- JOURNAL OF BIOLOGICAL CHEMISTRY vol. 263, 1988, pages 2442 - 2446 G.H. DAOUK ET AL.; 'Isolation of a functional human gene for brain creatine kinase'
- BIOCHEMICAL AND BIOPHYSICAL RESEARCH COMMUNICATIONS vol. 140, 1986, pages 981 - 989 M.B. PERRYMAN ET AL.;'Isolation and sequence analysis of a full-length cDNA for human M creatine kinase'
- JOURNAL OF CLINICAL INVESTIGATION vol. 83, 1989, pages 1637 - 1643 J.J. BILLADELLO ET AL.; 'Characterization of MB creatine kinase isoform conversion in virto and in vivo in dogs'
- CATHETERIZATION AND CARDIOVASCULAR DIAGNOSIS vol. 13, 1987, pages 26 - 32 A.M. GRACE ET AL.; 'Quantification of isoforms of plasma MM creatine kinase (CK) with an immunoblot procedure'

## Description

### Field of the Invention

The invention relates to DNA molecules coding for mutant subunits of creatine kinase, to recombinant vectors containing the DNA molecules, to host cells transformed with the vectors and to methods for producing mutant creatine kinase B and M subunits. The recombinant mutant and wild-type creatine kinase subunits of the invention form stable homo and heterodimers useful as analytical standards.

### Description of Related Art

Plasma creatine kinase (CK) enzyme analysis is of interest because it is currently the most cost-effective and specific method of diagnosing acute myocardial infarction as well as other muscle disorders. The basis of the analysis is the determination of several isoforms of CK which form *in vivo*, usually as dimers, form CK isozymes. The presence of different CK isoforms is generally an indication of muscle damage and is therefore valuable for diagnosis and treatment.

Five million patients with chest pain are evaluated annually in hospital emergency rooms each year in the United States and of these more than half are admitted to a coronary care unit. Yet less than 30% of patients with chest pain are later determined to have myocardial infarction (MI). The cost of care for patients admitted to a coronary care unit in whom MI is ruled out is approximately $4 billion per year (Selker, 1989). Patients in whom the diagnosis of MI is excluded are at low short-term risk for serious complications and could be managed safely in a less intensive setting.

However, while assays have been developed that detect elevated levels of MB CK dimers in patients having myocardial infarction within 6 hrs of symptom onset, there is often a problem in identifying all isoforms of creatine kinase particularly when electrophoretic analysis is used for identification.

A major concern in the routine use of the subforms of the creatine kinase MM isoenzyme (homodimeric form of the CK M subunit) as a diagnostic marker of myocardial injury is the lack of specificity: significantly increased concentrations of subform MM₁ (a posttranslationally modified homodimer of CK M subunit) in plasma are associated with skeletal muscle injury of diverse etiologies, including inflammatory disease and trauma associated with exercise. The presence of another subform of CK, an MB dimer, is associated with MI. While analysis of subforms of an MB isozyme of CK provides the necessary specificity, a significant problem with the analytical methods in current use is the lack of stable and reproducible isoform analytical standard markers.

Several factors contribute to misidentification of creatine kinase isoforms. At least four distinct human cDNAs encoding subunits of M creatine kinase, B creatine kinase, and two forms of mitochondrial creatine kinase (sarcomeric and ubiquitous) have been isolated and cloned. Association of the subunits leads to active dimeric and, at least for mitochondrial CK, octameric forms. Cardiac tissue contains high concentrations of the MM dimer, heterodimers MB CK, and octameric forms. The dimers BB CK and MM CK have been found in the human brain.

The isoforms, MM, BB, and the various dimers and octameric forms of sarcomeric and ubiquitous mitochondrial CK are thought to constitute the tissue forms of the enzyme; however, several distinct biochemical processes result in the production of variants of these species. Heterodimers of the mitochondrial CK isoforms and M CK and B CK have been demonstrated *in vitro.* These isoforms exhibit intermediate mobility following non-denaturing electrophoresis, adding to the complexity of interpretation of bands of CK activity after electrophoresis.

Yet another complication of CK analysis is the production of variants of subunit M CK arising from proteolytic cleavage of C-terminal lysine. This results in formation of numerous CK subforms, including dimeric species such as MM₁, MM₂ and MM₃ and subforms arising from combinations with CK B subunit, such as MB₁ and MB₂. The time course of these subforms has recently been used to facilitate early diagnosis of myocardial infarction, now detectable as early as four hours post infarction.

Generation of multiple electrophoretic variants can also arise from other types of *in vivo* post-translational modification, such as phosphorylation or glycosylation. These covalent modifications may result in altered electrophoretic mobility and appearance of new bands of CK activity.

Standard preparations of creatine isoforms are in demand as standards for analytical procedures to measure the various isoforms in human plasma. Widely used electrophoretic methods utilize commercial creatine kinase standard preparations which unfortunately have inherent problems of stability. Such preparations have relatively short shelf life and, of greater concern, often produce spurious bands during electrophoresis.

Creatine kinase isoforms are currently obtained from animal tissues and require multistep procedures for isolation. Tissue is used because once in the blood, creatine kinase isoforms are exposed to serum proteases resulting in significant degradation. Skeletal or heart muscle is the most commonly used source of creatine isoforms. Isolation procedures generally include at least two column chromatographies to obtain subforms of creatine kinase, *e.g.*, MM dimers. These dimers are then incubated with carboxypeptidase to obtain an isoform designated MM₁. Numerous additional steps are needed to produce other isoforms of MM, such as MM₂ and MM₃. Mixed isoforms, including those from creatine B subunit, *e.g.,* MB₂ or BB₁, require even more steps, including denaturation and dialysis. The procedures are time-consuming, often requiring several days.

An even further disadvantage of having to isolate creatine kinase isoforms from tissue is the need to use human rather than animal tissue when analytical standards for human isoforms are desired. The creatine kinase isoforms found in animal tissue are not suitable for standards in detection methods of human creatine kinase isoforms because there are subtle electrophoretic differences in CK isoforms from different species.

Recently, the availability of human tissue has been severely curtailed because of potential contamination with the AIDS virus. Additionally, suppliers are increasingly aware of potential liability in providing human tissue that may be contaminated with the AIDS virus, particularly without means of certifying the tissue as disease-free.

There is therefore a need to develop processes to produce CK isoforms from non-animal sources. Present methods pose threats of contamination and are inefficient and costly. In addition, isoforms of CK isolated from tissue sources have a relatively short shelf life and often give spurious results when employed as analytical standards.

### SUMMARY OF THE INVENTION

The present invention addresses one or more of the foregoing problems by providing methods to efficiently and rapidly produce creatine kinase isoforms *in vitro*. In relatively short periods of time, appropriately transfected cells provide good yields of creatine kinase isoforms that show good stability. The products expressed in the cells require little purification and generally may be isolated from lysed cells after separation from cell debris.

Mutant creatine kinase subunits are also part of the present invention and include lys⁻M and lys⁻B creatine kinase subunits. When DNA encoding the mutant CK subunits is incorporated into expression vectors, the mutant CK subunits are expressed in transfected host cells as stable homodimers. When suitable host cells such as COS cells are cotransfected with DNA encoding mutant B, mutant M, wild-type M or wild-type B subunits, the expressed products include homo and heterodimers of the CK subunits. The dimeric products exhibit identical electrophoretic mobilities with the major human creatine kinase isoforms which have been identified with pathological conditions such as myocardial infarction. Unlike human creatine kinase isoform standards isolated from human tissue, the isoforms expressed from recombinant cells are stable and show excellent reproducibility as standards in nondenaturing electrophoresis.

*In vivo* subforms of subunit M creatine kinase arise from posttranslational alterations in the plasma. The major event appears to be removal of a terminal lysine from CK subunit M. Resultant dimeric isoforms are conventionally designated MM₃, MM₂ and MM₁, representing dimers of wild-type M/wild-type M, wild-type M/lys⁻M and lys⁻M/lys⁻M and respectively. As used herein, the lys⁻ represents a form of creatine kinase lacking a carboxy terminal lysine. A similar convention applies to CK subunit B isoforms formed from various combinations of wild-type CK subunit B and its posttranslationally modified lys⁻ form. The tissue form of MB heterodimer is designated MB₂, representing a heterodimer formed from wild-type B and wild-type M. The plasma heterodimer is MB₁ CK formed from wild-type B and lys⁻M.

The mutant DNA molecules encoding CK subunits differ from DNA encoding wild-type CK subunits in having an altered nucleic acid sequence at the 3' terminus. When C is replaced with G, the lysine codon is changed to a stop codon, and the expressed polypeptide lacks a carboxy terminal lysine. Lys⁻M and lsy⁻B CK subunits may be obtained in this manner.

In one aspect, the invention may be understood as a novel method of rapidly producing recombinant CK subunit homo and heterodimers in transformed cells. The method typically involves transfection of appropriate host cells with a recombinant vector containing a CK subunit DNA sequence. When the vector contains CK M subunit DNA, the M subunit is expressed which then associates with another wild-type M subunit to form MM₃. Transfection of cells with a DNA encoding a mutant creatine kinase subunit lys⁻M will express CK M subunit with subsequent formation of MM₂ dimers. Cotransfection of cells with DNA encoding wild-type recombinant creatine kinase M subinit vectors and mutant creatine kinase M subunit vectors produces dimeric products MM₁, MM₂ and MM₃ understood to represent mutant/mutant, wild type/mutant and wild-type/wild-type dimeric forms of CK subunit M respectively. Cotransfection with expression vectors encoding creatine kinase M, mutant creatine kinase lys⁻M and creatine kinase B results in expression of CK subunits M, B and lys⁻M with formation of at least six dimeric species, including MM₁, MM₂, MM₃, MB₁,MB₂ and BB creatine kinase isoforms.

Recombinant creatine kinase subunit products lacking a carboxy terminal lysine residue are also within the ambit of the invention. Once expressed, the polypeptide subunits form stable dimers, homodimers with each other or heterodimers with ordinary wild-type CK subunits when cotransfection is with an expression vector encoding a wild-type subunit. The invention has been illustrated with CK M and B subunits but other CK subunits, including sarcomeric and ubiquitous mitochondrial creatine kinase, may also be produced. Mutant subunits, for example lys⁻M CK subunit, form stable dimers with each other and with wild-type subunits, typically exhibiting electrophoretic mobility on non-denaturing gel electrophoresis the same as mobility of wild-type counterparts. CK isoforms produced by the disclosed method are excellent analytical standards for determining CK isoforms, particularly when nondenaturing electrophoresis is employed.

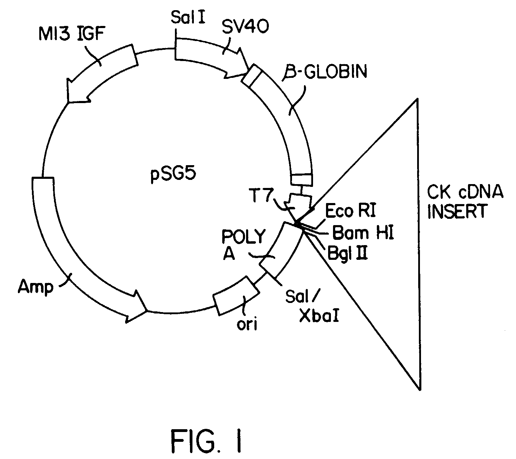

The recombinant DNA molecules of the present invention may be readily adapted for expression vectors. Any of a number of vectors may be constructed, including the cloning vector shown in Figure 1, that is, pSG5. In this preferred embodiment, a creatine kinase cDNA is inserted into the *Eco*RI site, employing methods well known to those of skill in the art.

Turning firstly to the expression of the mutant CK subunits. Once a suitable (full length if desired) clone or clones have been obtained, whether they be cDNA based or genomic, one may proceed to prepare an expression system for the recombinant preparation of any subunit of CK. The engineering of DNA segment(s) for expression in a prokaryotic or eukaryotic system may be performed by techniques generally known to those of skill in recombinant expression. It is believed that virtually any expression system may be employed in the expression of CK subunits.

Creatine kinase subunits have been successfully expressed in eukaryotic expression systems with the production of active enzyme, however, it is envisioned that bacterial expression systems may ultimately be preferred for the preparation of large amounts of CK isoforms. The cDNA for CK subunits may be separately expressed in bacterial systems, with the encoded proteins being expressed as fusions with β-galactosidase, ubiquitin, glutathione S-transferase, and the like. It is believed that bacterial expression will ultimately have numerous advantages over eukaryotic expression in terms of ease of use and quantity of materials obtained thereby.

Transformation of host cells with DNA segments encoding the CK subunits B or M provides a convenient means for obtaining active enzyme. However, separate expression followed by reconstitution is also certainly within the scope of the invention. Both cDNA and genomic sequences are suitable for eukaryotic expression, as the host cell will, of course, process the genomic transcripts to yield functional mRNA for translation into protein.

It is similarly believed that almost any eukaryotic expression system may be utilized for the expression of CK subunits, whether wild-type or mutant, *e.g.,* baculovirus-based, glutamine synthetase-based or dihydrofolate reductase-based systems could be employed. However, in preferred embodiments, the cloning vector of Figure 1 may be employed. Plasmid vectors incorporating an origin of replication and an efficient eukaryotic promoter, as exemplified by the eukaryotic vectors of the pCMV series, such as pCMV5, may also be of use.

For expression in this manner, one would position the coding sequences adjacent to and under the control of the promoter. It is understood in the art that to bring a coding sequence under the control of such a promoter, one positions the 5' end of the transcription initiation site of the transcriptional reading frame of the protein between about 1 and about 50 nucleotides "downstream" of (i.e., 3' of) the chosen promoter.

In eukaryotic systems, one also will typically desire to incorporate into the transcriptional unit which includes the enzyme, an appropriate polyadenylation site (e.g., 5'-AATAAA-3') if one was not contained within the original cloned segment. Typically, the poly A addition site is placed about 30 to 2000 nucleotides "downstream" of the termination site of the protein at a position prior to transcription termination.

As noted above, it is proposed that in embodiments concerning the production of CK subunits, the M and B subunits may be co-expressed in the same cell. This may be achieved by co-transfecting the cell with two distinct recombinant vectors, each bearing a copy of either the CK B subunit or CK M subunit-encoding DNA. The same holds true for cotransfection with three or more different vectors.

Alternatively, a single recombinant vector may be constructed to include the coding regions for all of the subunits, which could then be expressed in cells transfected with the single vector. Several variations of these vectors would be possible depending on the relative positions of the different CK subunit DNA. Expression efficiencies might depend on the relative positions.

In either event, whether single or multiple expression vectors are employed, the term "co-expression" herein refers to the expression of two or more subunits of creatine kinase in the same recombinant cell. It should be noted, however, that should one desire to obtain separate polypeptide subunits, it will be convenient to construct vectors with different promoters for each creatine kinase coding segment, resulting in same-cell expression of the different subunits.

As used herein, the term "engineered" or "recombinant" cell is intended to refer to a cell into which a recombinant gene, such as a gene encoding a mutant CK subunit has been introduced. Therefore, engineered cells are distinguishable from naturally occurring cells which do not contain a recombinantly introduced gene. Engineered cells are thus cells having a gene or genes introduced through the hand of man. Recombinantly introduced genes will either be in the form of a cDNA gene (i.e., they will not contain introns), a copy of a genomic gene, or will include genes positioned adjacent to a promoter not naturally associated with the particular introduced gene.

Generally speaking, it may be more convenient to employ as the recombinant gene a cDNA version of the gene. It is believed that the use of a cDNA version will provide advantages in that the size of the gene will generally be much smaller and more readily employed to transfect the targeted cell than will a genomic gene, which will typically be up to an order of magnitude larger than the cDNA gene. However, the inventors do not exclude the possibility of employing a genomic version of a particular gene where desired.

As mentioned above, modification and changes may be made in the structure of CK subunit and still obtain a molecule having like or otherwise desirable characteristics. For example, certain amino acids may be substituted for other amino acids in a protein structure without appreciable loss of interactive binding capacity with structures such as, for-example, antigen-binding regions of antibodies or binding sites on substrate molecules. Since it is the interactive capacity and nature of a protein that defines that protein's biological functional activity, certain amino acid sequence substitutions can be made in a protein sequence (or, of course, its underlying DNA coding sequence) and nevertheless obtain a protein with like or even countervailing properties (e.g., antagonistic v. agonistic). It is thus contemplated by the inventors that various changes may be made in the sequence of creatine kinase subunit polypeptide (or underlying DNA) without appreciable loss of their biological utility or activity.

In making such changes, the hydropathic index of amino acids may be considered. The importance of the hydropathic amino acid index in conferring interactive biologic function on a protein is generally understood in the art (Kyte et al., J. Mol. Biol., 157:105-132, 1982). It is known that certain amino acids may be substituted for other amino acids having a similar hydropathic index or score and still retain a similar biological activity. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is believed that the relative hydropathic character of the amino acid determines the secondary structure of the resultant protein, which in turn defines the interaction of the protein with other molecules, for example, enzymes, substrates, receptors, antibodies, antigens, and the like. It is known in the art that an amino acid may be substituted by another amino acid having a similar hydropathic index and still obtain a biological functionally equivalent protein. In such changes, the substitution of amino acids whose hydropathic indices are within ±2 is preferred, those which are within ±1 are particularly preferred, and those within ±0.5 are even more particularly preferred.

Substitution of like amino acids can also be made on the basis of hydrophilicity, particularly where the biological functional equivalent protein or peptide thereby created is intended for use in immunological embodiments. U.S. Patent 4,554,101, incorporated herein by reference, states that the greatest local average hydrophilicity of a protein, as governed by the hydrophilicity of its adjacent amino acids, correlates with its immunogenicity and antigenicity, i.e. with a biological property of the protein.

As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); (0 ± 1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those which are within ±1 are particularly preferred, and those within ±0.5 are even more particularly preferred.

Amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

Expression vectors containing DNA sequences according to the present invention are readily transfected into appropriate host cells that are capable of expressing the human kinase subunits. It is contemplated that virtually any of the commonly employed host cells can be used in connection with the expression of CK subunits in accordance herewith. Examples include cell lines typically employed for eukaryotic expression such as T3T, HepG2, 10T½, 239, AtT-20, VERO HeLa, CHO, WI38, BHK, RIN and MDK cell lines. A preferred line for use in eukaryotic expression embodiments of the present invention has been found to be the COS cell line because high expression of product creatine kinase subunits is obtained with low endogenous creatine kinase expression.

In a preferred aspect of the present invention, COS cells are co-transfected with recombinant creatine kinase M, recombinant CK B and recombinant mutant M subunits. Formation of heterodimeric products of creatine kinase is readily detected. These dimers are formed from M or B subunits and mutant M subunits as well as homodimeric forms such as two wild-type subunits of creatine kinase or mixed dimeric species of M and B wild-type subunits. Whether co-transfection is with two or more recombinant DNA vectors, stable isoforms of creatine kinase may be produced in the host cells. Co-transfection is of course not limited to these particular recombinant forms of creatine kinase. Various mutations of creatine kinase subunit M, creatine kinase subunit B, as well as variations of sarcomeric mitochondrial creatine kinase and ubiquitous mitochondrial creatine kinase are expected to produce a wide variety of homo- and heterodimeric forms of creatine kinase.

A wide variety of stable isoforms of creatine kinase are therefore produced by transfecting host cells with expression vectors as herein described. Host cells are incubated in appropriate media and harvested to obtain stable isoforms of creatine kinase. Depending on the particular range of products desired, particularly for dimeric species, co-transfection with two or more of the described expression vectors containing the recombinant DNA sequence may be employed. Stabilized forms may be isolated from cells disrupted by any of a number of techniques, including sonication, detergent extraction, mechanical homogenization, and repeated freeze-thawing. In practice of the present invention, it has been found that removing cell debris from the lysate and directly utilizing the supernatant or filtrate depending on the method of removal, provides a stable composition of CK isoforms.

It is not intended that the invention should be limited to methods of producing only the particular mutant CK subunits described. It is contemplated that numerous changes may be made in DNA molecules encoding CK subunits having substantially the same properties as the mutant lys⁻M subunit. Thus, alteration of the CK DNA sequence may result in certain amino acids substituted for other amino acids without substantial alteration in activity or function, as previously mentioned.

Yet another aspect of the invention is a composition that is useful as an analytical standard. Such a composition includes any of the creatine kinase isoforms obtained from creatine kinase subunit M, subunit B and mutant subunits of creatine kinase subunits M and B. Typically the isoforms will be dimeric subunits. However, other multimeric forms may be isolated. It is known that mitochondrial type creatine kinase M will form octameric subunits *in vivo.* It is expected that these forms will be present in cell lysates from host cells transfected with mutant creatine kinase expression vectors and/or cotransfected with one or more of these vectors.

The invention also includes compositions comprising any of creatine kinase isoforms that arise from association of creatine kinase M subunit, creatine kinase B subunit, sarcomeric mitochondrial creatine kinase subunits and ubiquitous mitochondrial creatine kinase subunits. Any of these species could be expressed individually or co-expressed with each other from a host cell, for example, from COS cells. Stable mutations analogous to that described for CK subunit M should also be appropriate for sarcomeric mitochondrial and ubiquitous creatine kinase.

The compositions of the present invention are particularly useful as analytical standards. In most preferred practice, these species are employed as stable reproducible standards in non-denaturing electrophoresis. Numerous and various isoforms available in the dimeric form particularly show small but reproducible and distinct differences on nondenaturing electrophoresis and are valuable for identification of normal and pathogenic isoforms in human diagnostics. It is recognized, for example, that certain isoforms may be found in-human serum or plasma shortly after myocardial infarction. This has led to more reliable methods of diagnosing myocardial infarction in human patients. By analogy, it is contemplated that other forms, abnormal or otherwise, of sarcomeric mitochondrial creatine kinase, ubiquitous mitochondrial creatine kinase and perhaps other forms of creatine kinase M or B subunits may be employed in future diagnostic procedures to identify pathological states of brain chemistry or damage and/or muscle injury.

The stable isoforms of creatine kinase herein demonstrated have been illustrated with a single mutation of creatine kinase subunit M. However, it will be appreciated by those of skill in the art that analogous mutations could be conducted in virtually any part of the creatine kinase M subunit or the creatine kinase B subunit to produce analogous.isoforms with an expectation of increased stability. Additionally, mutations need not be limited to point mutations but could include substitutions and or deletions. Cotransfection of appropriate host cells with analogously constructed expression vectors would be expected to produce similar isoforms of creatine kinase. Similar considerations would be involved in the development of recombinant expression vectors for creatine kinase subunits of sarcomeric mitochondrial creatine kinase and ubiquitous mitochondrial creatine kinase. Stable isoforms of mitochondrial creatine kinase dimers may be of importance in developing even more sensitive methods of detection of aberrations involving creatine kinase forms.

In general, the technique of site specific mutagenis is well known in the art as exemplified by publications (Adelman, *et al.,* 1983). As will be appreciated, the technique typically employs a phage vector which exists in both a single stranded and double stranded form. Typical vectors useful in site directed mutagenesis include vectors such as the M13 phage (Messing *et al.* 1981). These phage are readily commercially available and their use is generally well known to those skilled in the art.

Site directed mutagenesis in accordance herewith is performed by first obtaining a single stranded vector which includes within its sequence the DNA sequence encoding a subunit of creatine kinase. An oligonucleotide primer bearing the desired mutated sequence is prepared, generally synthetically, for example, by the method of Cray, *et al.* (1978). The primer is then annealed with the single stranded vector and subjected to DNA polymerizing enzymes such as the *E. coli* polymerase 1 Klenow fragment. In order to complete the synthesis of the mutation bearing strand, thus, a heteroduplex is formed wherein one strand encodes the original non-mutated sequence and the second strand bears the desired mutation. The heteroduplex is subcloned back into the vector and the vector containing the mutated insert is then used to transform appropriate cells such as COS cells. Clones are selected which include recombinant vectors bearing the mutated sequence arrangement.

The preparation of sequence variance of the selected exportation polypeptide gene using site directed mutageneses is provided as a means of producing creatine kinase subunit mutants and is not meant to be limiting as there are other ways in which sequence variance of CK subunit genes may be obtained. For example, recombinant vectors encoding the desired CK gene subunits may be treated with mutagenic agents to obtain sequence variance (see, *e.g.,* a method described by Eichenlaub (1979) for the mutagenesis of a plasmid DNA using hydroxylamine).

Kits useful for the expression of various creatine kinase subunits are also envisioned as part of the invention. Such kits would include separate containers each having suitably aliquoted reagents for performing the foregoing methods. For example, the containers may include one or more expression vectors prepared in accordance with claim 6. A preferred cloning vector, shown in Figure 1, is employed for insertion of a desired cDNA, *e.g.,* subunit M CK DNA, at the *Eco*RI site. Suitable containers mighty be vials made of plastic or glass, various tubes such as test tubes, metal cylinders, ceramic cups or the like. Containers may be prepared with a wide range of suitable aliquots depending on applications and on the scale of the preparation. Generally this would be an amount that is conveniently handled so as to minimize handling and subsequent volumetric manipulations. Most practitioners will prefer to select a cell line such as COS although other cell lines may be used for transfection with the appropriate vectors.

Vectors supplied in kit form are preferably supplied in lyophilized form although such DNA fragments may also be taken up in a suitable solvent such as ethanol, glycols or the like and supplied as extensions. For most applications, it would be desirable to remove solvent which for ethanol, for example, is relatively simple matter of evaporation.

### BRIEF DESCRIPTION OF THE DRAWINGS

Figure 1 shows the plasmid construct in which cDNA for creatine kinase was inserted.

Figure 2 shows the nondenaturing electrophoresis of creatine kinase isoforms and subforms. COS cells were cotransfected with three separate constructs: M creatine kinase, mutant creatine kinase M and B creatine kinase. Panel A shows three creatine kinase isoforms, MM, MB and BB. In Panel B, MM₁, MM₂ and MM₃ are detected as indicated.

Figure 3 shows the effect of storage temperature on creatine kinase enzyme activity.

Figure 4 is the nucleic acid sequence of human creatine kinase subunit B (SEQ ID NO.1).

Figure 5 is the nucleic acid sequence of human creatine kinase subunit M (SEQ ID NO 2). Mutant lys⁻M CK has a T in place of A at position 1219.

Figure 6 shows agarose gel electrophoresis of extracts from COS cells cotransfected with M and B creatine kinase cDNAs. The cell extract in lanes 1-6 are from cells transfected with 20 µg of M creatine kinase cDNA and increasing the B creatine kinase cDNA from 0 µg to 25 µg in increments of 5 µg. Lanes 7-9 show the results when the B creatine kinase construct is transfected in a concentration of 20 µg and the M creatine kinase cDNA concentration is increased from 0 µg to 25 µg in 5 µg increments.

### DETAILED DESCRIPTION OF PREFERRED EMBODIMENTS

The invention concerns novel DNA molecules representing mutant forms of creatine kinase subunits and recombinant vectors constructed from the DNA. Host cells transfected with the novel recombinant vectors-herein described are useful in efficiently producing a variety of stable CK dimeric isoforms that have application as analytical standards. The novel methods of producing CK isoforms in host cells avoid the use of human tissue in producing the isoforms and are much more efficient than procedures in current use.

The invention demonstrates construction of a recombinant mutant of human M creatine kinase. The mutant creatine kinase isoform is readily expressed from transfected COS cells. When COS cells are cotransfected with human M creatine kinase and the mutant creatine kinase, heterodimers readily form. The dimers are designated as MM₁, MM₂ and MM₃. They correspond to three CK M subunit dimers formed *in vivo* but may differ slightly in electrophoretic mobility in some cases where posttranslational changes in addition to lysine cleavage have occurred. The major advantages of the disclosed dimers are significantly increased stability, long term storage, and ready detectibility on commercially available electrophoretic analysis systems.

Transient infection of COS cells with full length human creatine kinase and B creatine kinase cDNAs inserted into the pSG5 plasmid demonstrates that enzymatically active proteins are expressed. Expression of creatine kinase activity has been detected 16 hours following transfection. This increases in a linear fashion through 48 hours post transfection. Specific activity of the human M creatine kinase is about two units per milligram of protein which is about one tenth the amount of creatine kinase activity found in heart tissue. The electrophoretic mobility of creatine kinase isoforms present in the COS cells following transfection was demonstrated by non-denaturing electrophoresis. These recombinant proteins have the same electrophoretic mobility as M creatine kinase purified from the human heart. Their mobility is unchanged after preincubation in human serum which is devoid of creatine kinase activity. Control transfections using the reverse orientation in the same plasmid have shown no M creatine kinase enzyme activity.

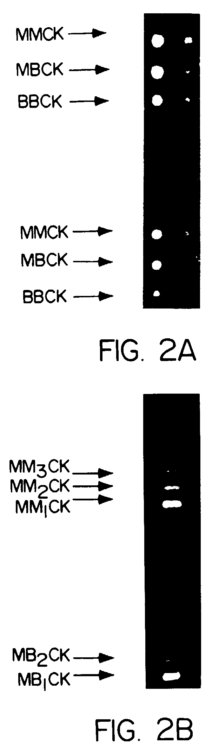

When constructs for creatine kinase M, B and lys⁻M subunits were cotransfected in COS cells, formation of heterodimeric MB creatine kinase was detected in addition to various MM creatine kinase and BB creatine kinase isoforms (Figure 2).
Cotransfection of the mutant MM creatine kinase minus the N-carboxy terminal lysine and the BB creatine kinase constructs also produced heterodimeric MB creatine kinase. This hybrid had an electrophoretic mobility slightly more anodic than wild type MB creatine kinase corresponding to the mobility difference observed for MM creatine kinase mutant compared with wild type MM creatine kinase.

When all three constructs were cotransfected, electrophoretic differences were easily detected, as shown in Figure 2. In line 3, five bands of creatine kinase activity were detected: these included MM₁ creatine kinase, MM₂ creatine kinase, MB₃ creatine kinase, MB₂ creatine kinase and BB creatine kinase. The mobility differences for the MB creatine kinase subforms MB₃ creatine kinase and MB₂ creatine kinase were more easily detected using the REP system, as shown in Figure 2, Panel B. The electrophoretic mobilities were unchanged when the cell extract was mixed with an equal volume of serum prior to electrophoresis.

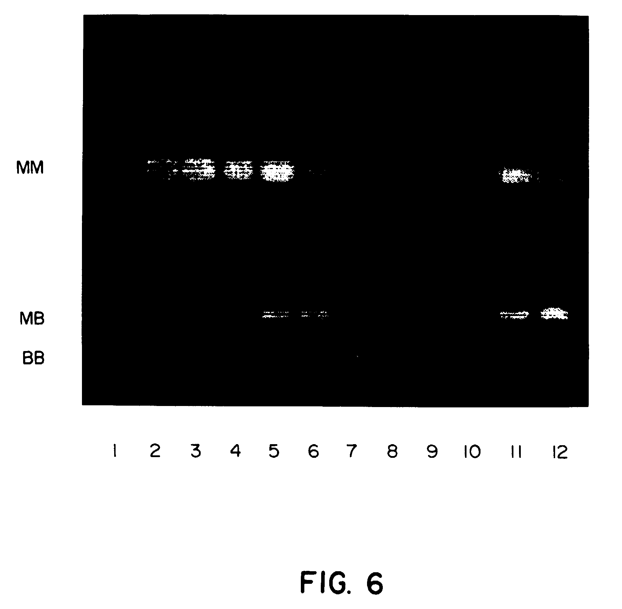

The formation of the MB creatine kinase heterodimer apparently was dependant upon the level of expression of the M creatine kinase and B creatine kinase mRNAs within the same cell. The formation of the heterodimer MB creatine kinase was dependant upon the relative proportion of each DNA construct used for the cotransfections. When the M creatine kinase construct was used in excess and the B creatine kinase construct was added in increasing amounts there was a parallel increase in the formation of heterodimeric MB creatine kinase (Figure 6). When the reverse experiment was carried out, B creatine kinase increased in parallel with the amount of M creatine kinase construct added.

Electrophoretic analysis of serum creatine kinase isoform content is currently widely used for the diagnosis of myocardial infarction and skeletal muscle disease. An important factor contributing to the misidentification of creatine kinase isoforms by nondenaturing electrophoretic methods is the lack of complete understanding of the number of creatine kinase isoforms and the precise tissue distribution. At least four distinct human cDNAs encoding subunits of M creatine kinase, B creatine kinase, ubiquitous mitochondrial creatine kinase and the sarcomeric mitochondrial creatine kinase have been isolated and cloned. It is generally accepted that enzymatically active creatine kinase consists of the dimeric or octameric association of subunits (Schlegel *et al.* 1988). Heterodimers of the M creatine kinase and B creatine kinase subunits, MB, are found in cardiac tissue and forms the basis of detection of myocardial infarction by electrophoretic methods. High concentrations MM creatine kinase are also present in human cardiac tissue. Sarcomeric mitochondrial creatine kinase in the dimeric and octameric forms has also been reported.

These five isoforms namely, MM, MB, BB and dimers of ubiquitous and sarcomeric mitochondrial creatine kinase constitute the tissue forms of the enzyme. However, several distinct biochemical processes result in the production of electrophoretic variance of these five isoforms. These isoforms exhibit intermediate mobility following nondenaturing electrophoresis, adding to the complexity of interpretation of bands of creatine kinase activity following nondenaturing electrophoresis.

Multiple electrophoretic variance of M creatine kinase also results from proteolytic cleavage of the C terminal lysine. This results in formation of MM creatine kinase subforms MM, M₁M and M₁M₁. Two creatine kinase subforms of MB (MB₁ and MB₂) are also found. The time course of the appearance of these subforms has been used to facilitate early diagnosis of myocardial infarction which can now be detected as early as four hours post MI (Puleo *et al.* 1991).

The generation of multiple electrophoretic variance can also result from other types of post-translational modifications. Covalent modification by phosphorylation (Chida *et al.* 1990) or glycosylation (O'Brien *et al.* 1991). Such modifications result in altered electrophoretic mobility in the appearance of novel bands of creatine kinase activity.

The creatine kinase isoforms disclosed as part of the present invention have enabled the development of a defined set of creatine kinase standard proteins which are exceptionally stable. These recombinant proteins have been characterized with respect to their electrophoretic mobility and shown to be useful in at lease two different gel systems commonly employed for nondenaturing electrophoresis in the clinical analysis of creatine kinase isoform compositions.

The following examples are intended to illustrate the practice of the present invention and are not intended to be limiting. Although the invention has been illustrated with a particular mutant of the M subunit of CK, other mutations, such as point mutations, deletions, etc. could be employed to provide suitable subunits. Subunits may be employed as monomers or dimeric species.

### EXAMPLE 1

This example illustrates construction of vectors containing the human M and human B creatine kinase subunits and a mutant creatine kinase B subunit.

### Insertion of cDNAs into Expression Vectors

### M CK and B CK Subunits

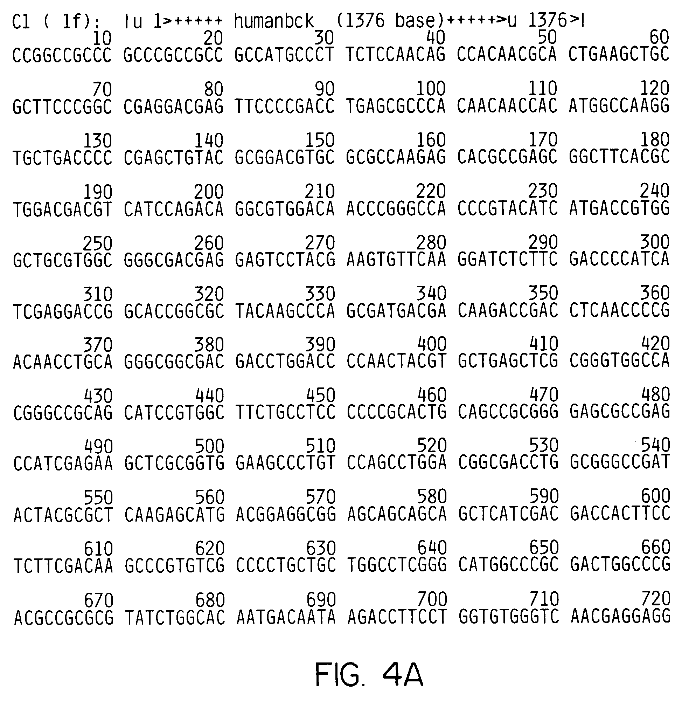

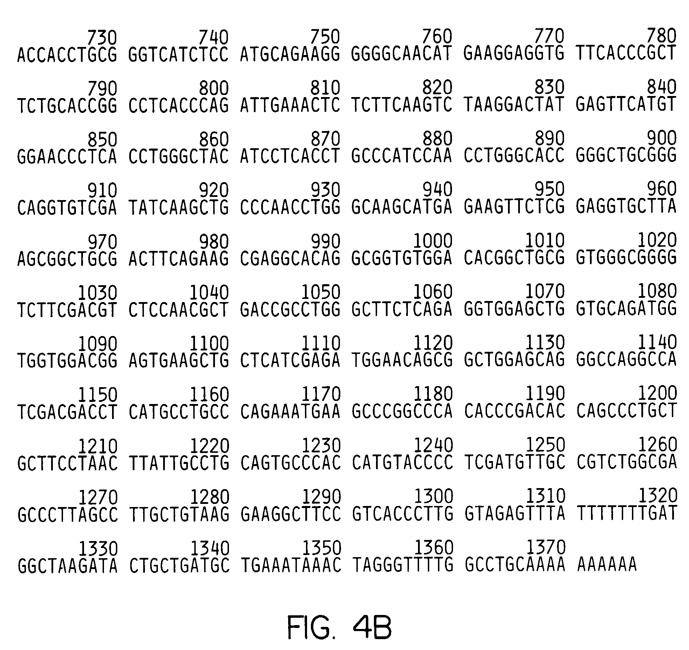

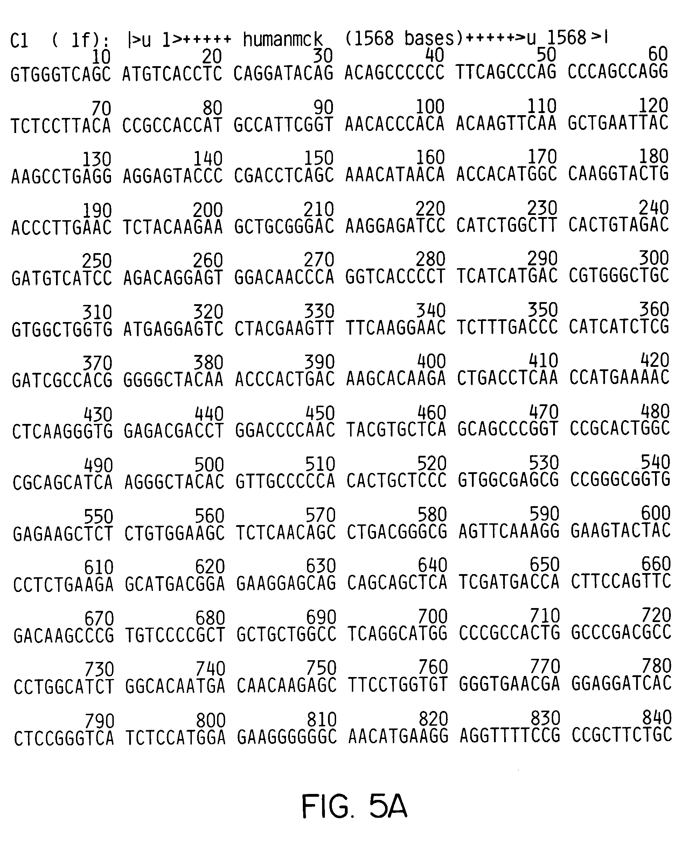

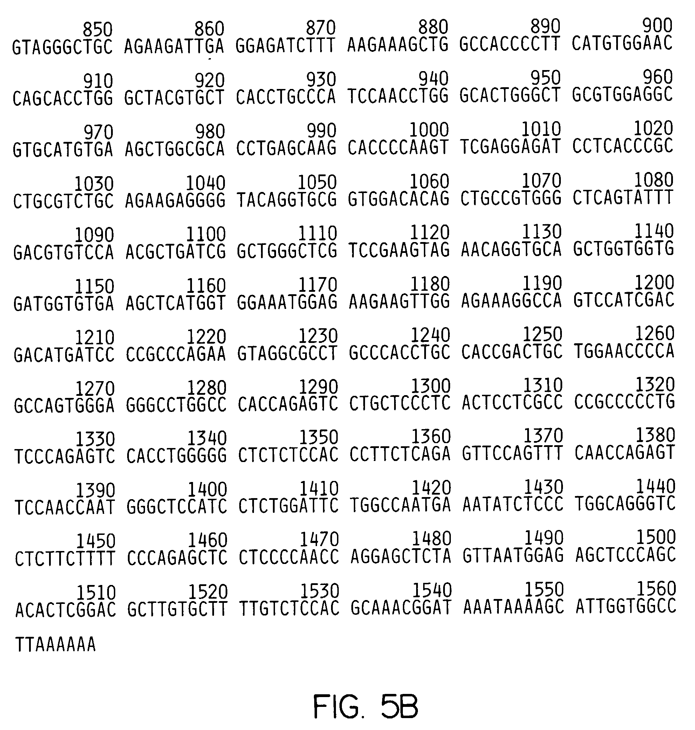

Full length human M creatine kinase and B creatine kinase cDNAs were used as templates for polymerase chain reaction amplification using oligonucleotide primers with *Eco*RI endonuclease recognition sites at the 5' ends. The amplified inserts were electrophoresed on low melting point agarose gels, isolated from the agarose, digested with *Eco*RI and inserted into the *Eco*RI site of the eukaryotic expression, pSG5 (Stratagene). The constructs were sequenced to confirm the orientation. Nucleotide sequences of human B CK and M CK subunits are shown in Figures 4 and 5 respectively.

### Mutant Human M CK Subunit (lys⁻M)

To produce mutant creatine kinase subunit lys⁻M, an oligonucleotide primer was synthesized with a single nucleotide mismatch in the codon for the carboxy terminal lysine residue of M creatine kinase. The sequence for the oligonucleotide primer which converts the terminal lysine codon to a stop codon was 5'-TAACCATTATAAGCTGCAATAAACAAGTTC-3'. This primer also contains an *Eco*RI endonuclease recognition sites at the 5' end. The wild-type M CK construct was used as template and PCR amplification was carried out using a T7 primer and the mutagenic M CK primer. The PCR reactions were carried out using 1 µM each primer, 1 µg template DNA, 2.5 U Taq DNA polymerase, 200 µM dNTPs and reaction buffer in a final volume of 100 µl. The reaction was carried out for 35 cycles at 1 min at 95°C, then 2 min at 55°, and then for 3 min at 72°C. The resulting PCR product was purified, restricted with *Eco*RI, recloned into pSG5, sequenced to determine orientation, and used in transfection of appropriate host cells.

The mutant lys⁻M CK subunit differs from wild-type human M CK subunit by a single base substitution at position 1219, shown in the sequence of Figure 5. The lysine codon, AAG, has been converted to TAG (position 1219-1221) which acts as a stop codon. Expressed mutant M CK protein differs from wild-type M CK subunit due to lack of a carboxy terminal lysine group.

### EXAMPLE 2

Transfection of COS cells is illustrated in this example. COS cells were chosen because activity of endogenous BB creatine kinase is low while MM isozyme and mitochondrial creatine kinase activity is undetectable.

### COS Cell Transfection

COS cells were cultured in DMEM supplemented with 10% fetal calf serum. The cells were plated the day prior to transfection at a density of 0.6 x 10⁶ cells per 60 mm dish. The DNA was introduced into the cells using the DEAE dextran method (Razzaque, *et al.,* 1982), although lipofection was also found suitable for these cells. At 48 hr post-transfection, the cells were harvested and the creatine kinase activity assayed and the isoenzyme content determined using agarose gel electrophoresis. The DNA (10 µg) and DMEM media were combined, then the DEAE dextran was added in order to avoid precipitation of the DNA. The mixture was added to the cells (1.5 ml/60 mm dish) and incubated at 37°C for 3 hrs. The media was then removed and the cells were shocked with 10% DMSO prepared fresh, for 60 seconds. The cells were rinsed and then replenished with DMEM containing 10% FCS. The cells were harvested at various time points following the transfections using a detergent lysis buffer containing 2% NP-40, 50 mM Tris-HCl (pH 8.3), and 5 mM BME. The extract was centrifuged at 12,000 x g for 10 minutes and the supernatant was used without further purification for enzyme analysis.

Cotransfection with a neomycin resistance gene provided a convenient method to select for stable transformants. Resistance plaques selected with mitomycin were used for the selection.

Co-transfection with M and B creatine kinase subunit constructs resulted in production of all three cytoplasmic creatine kinase isoenzymes with a specific activity of 2.0 IU/mg. Triple co-transfection of COS cells with the M, lys⁻M and B constructs resulted in the production of all three MM and both MB creatine kinase subforms as well as BB creatine kinase at a specific activity of 2.0 IU/mg. Agarose gel analysis of the creatine kinase expression products are shown in Figure 2.

### EXAMPLE 3

Activity and stability of various subforms of CK dimeric species were tested. This example illustrates the long term stability of CK isoforms stored at 4°C.

### Creatine Kinase Specific Activity Determinations

Tissue extracts were analyzed for creatine kinase activity in the direction of ATP synthesis employing a coupled enzyme assay with hexokinase and glucose 6 phosphate dehydrogenase. Detection was based on the reduction of NADPH measured spectrophotometrically (Rosalki, 1966). Units were defined as picomoles creatine phosphate hydrolyzed per minute. Protein determinations were carried out using a modified Lowrey assay and bovine serum albumin standard.

Stability of the MB construct was measured by adding a mixture of the MB and M₁B (lys⁻MB) cell cultures to a composition of 50 mmol/L Tris buffer, pH 7.2, 30 g per l immunopure bovine serum albumin, 2 mmol/L adenosine diphosphate, 10 mmol/L N-acetyl cystine, 5 mmol/L ethylenediamine tetraacetic acid, and 0.1% sodium azide (Baskin and Deamer, 1970). The stability of the composition was compared against two other compositions 1) a commercial CK-MB control (i.d. zone, Beckman Instruments, Brea, CA) containing MM, MB (MB₂) and BB in a human serum albumin matrix stabilized with ethylene glycol and 2) a commercial MB (MB₂) material preparation of purified human myocardium (Alto Scientific, San Marcos, CA) diluted into the previously herein described Tris buffer. All preparations were allocated into separate vials and stored for two months at -20°C, 4°C, ambient room temperature, 37°C and 45°C.

When standards were frozen or stored at refrigerated temperatures, they were stable when employed for assays. However, degradation of the Beckman control material was evident for total CK when the standards were stored at room temperature. For MB, some loss of activity was noted in all samples stored at room temperature. In contrast, MB mass concentration remained constant at this temperature. When the storage temperature was increased to 37° and 45°C all materials were degraded when stored at the elevated temperatures.

### EXAMPLE 4

The following example shows the resolution of creatine kinase isoforms and subforms by nondenaturing electrophoresis.

### Electrophoresis of CK Isoforms Expressed in COS Cells

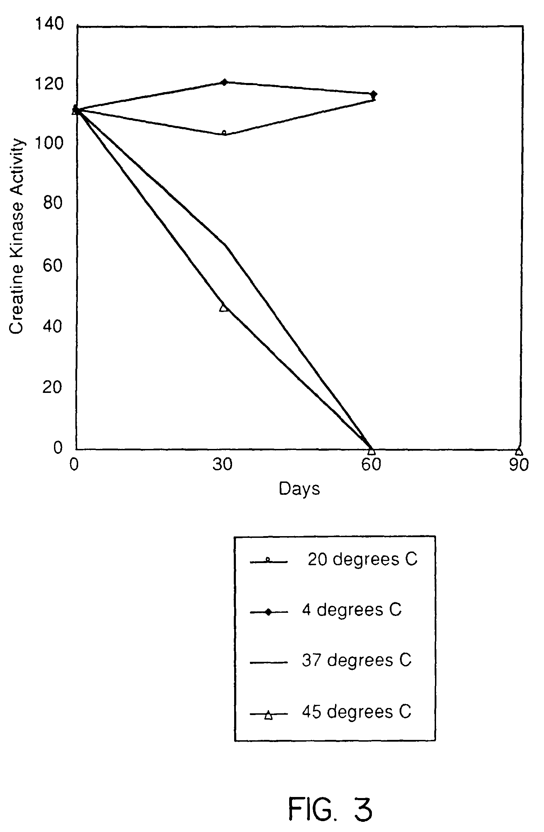

Detergent extracts of COS cells were resolved by nondenaturing electrophoresis using the REP system (Helena Labs) Rapid Electrophoresis System. The COS cells were transfected with subunit B, subunit M and mutant M recombinant expression vectors. Tissue extracts and purified standards (1 mm/l; 10 mu/gs) were loaded onto 1% agarose gels and run in Tris-barbital buffer at 175 v for 45 min at 4°C. The gels were overlaid with creatine kinase substrate (Rosalki reagent) and incubated for 15 min at 37°C. The reaction product was visualized with UV light results are shown in Figure 3. As shown in Panel A, all three creatine kinase isoforms, MM, MB and BB, were detected. In panel B the electrophoresis conditions were modified to maximize the resolution of the MM-CK and MB-CK isoforms. At the top of Panel B the MM-CK subforms are detected MM₁-CK, MM-CK, M₁M₁-CK. The anode is at the bottom of the figure and the cathode is at the top.

Total creatine kinase activity was measured using a Hitachi 717 Analyzer with CK-NAC reagents at 37°C (Boehringer Mannheim Diagnostics, Indianapolis, IN), MB activity using the DRI-STAT immunoinhibition assay adapted to the Cobas Bio Centrifugal Analyzer (Roche Diagnostics) MB mass concentration using the Stratus II (Baxter Healthcare Corporation, Miami, FL) and MB isoforms using high-voltage electrophoresis (REP, Helena Laboratories, Beaumont, TX).

As shown in Figure 2, the REP System provided a greater separation of MM and MB subforms of creatine kinase. COS cells were cotransfected with three constructs: M creatine kinase, M creatine kinase mutant (lys⁻M) and B creatine kinase. The three bands at the top of the gel represent MM creatine kinase, MM₁ creatine kinase and M₁M₁ creatine kinase. M₁ refers to the recombinant mutant subunit lacking in terminal lysine compared to the natural form. The two lower bands are the MB creatine kinase subforms MB and M₁B creatine kinase.

### REFERENCES

The references listed below are incorporated herein by reference to the extent that they supplement, explain, provide a background for or teach methodology, techniques and/or compositions employed herein.

Adelman, J.P., Hayflick, J.S., Vasser, M. and Seeburg, P.H., *DNA* **2/3**, 183-193 (1983).

Baskin, R.J., and Deamer, D.N. (1970) *J. Biol. Chem.* **245**, 1345-1347.

Benfield, P.A., Henderson, L., and Pearson, M.L. (1985) *Gene* **39**, 263-267.

Bessman, S.P. (1972) *Israel. J. Med. Sci*. **8**, 344-352.

Bessman, S.P., and Carpenter, C.L. (1985) *Ann. Rev. Biochem.* **54**, 831-862.

Eichenlaub, R. *J. Bacteriol.* **138**, 559-566 (1979).

Friedman, D.L., and Perryman, M.B. (1991) *J. Biol. Chem.* **266**, 22404-22410.

Hossle, J.P., Rosenberg, W.B., Schafer, B., Eppenberger, H.M., and Perriard, J.C. (1986) *Nucleic Acids Res.* **14**, 1449-1463.

Jacobs, H., Heldt, H.W., and Klingenberg, M. (1964) *Biochem. Biophys. Res. Commun.* **16**, 516-521.

Kyte, *et al., J. Mol. Biol* 157:105-132 (1982)

Lowry, O.H., Rosebrough, N.J., Farr, A.L., and Randall, R.J. (1951) *J. Biol. Chem.* **193**, 265-275.

Perryman, M.B., Knell, J.D., Ifegwu, J., and Roberts, R. (1985) *J. Biol. Chem.* **260**, 9399-9404.

Razzaque, A., Mizusawa, H., and Seidman, M.M. *Proc. Natl. Acad. Sci.* U.S.A. 80:3010.

Rossi, A.M., Eppenberger, H.M., Volpe, P., Cotrufo, R., and Walliman, T. (1990) *J. Biol. Chem.* **265**, 5258-5266.

Saks, V.A., Lipina, N.V., Sharov, V.G., Smirnov, V.N., Chazov, E., and Grosse, R. (1977) *Biochem. Biophys. Acta* **465**, 550-558.

Saks, V.A., Rosenshtraukh, L.V., Elizarova, G.V., and Jacobus, W.E. (1978) *Can. J. Physiol. Pharmacol.* **56**, 691-706.

Savabi, F., Geiger, P.J., and Bessman, S.P. (1983) *Biochem. Biophys. Res. Commun.* **114**, 785-790.

Scholte, H.R., Weijers, P.J., and Wit-Peeters, E.M. (1973) *Biochim. Biophys. Acta* **291**, 764-763.

Selker, H.P. "Coronary Care and Triage Decision Aids-How Do We Know When They Work?" *Am. J. Med.* 87, 491-493 (1989).

Whitner, V.S., Morin, L.G., McKneally, S.S., Sampson, E.J. Stability of creatine kinase-3 in lyophilized materials. *Clin Chem* (1982) **28**, 41-44.

Puleo, P.R., Guadagno, P.A., Roberts, R., Perryman, M.B. Sensitive, rapid assay of subforms of creatine kinase MB in plasma. *Clin Chem* (1989) **35,** 1452-1455.

U.S. Patent No. 4,554,101, November 19, 1985, Hopp, T.P.

## Claims

1. A DNA molecule comprising a coding sequence for a mutant creatine kinase M or B subunit which is stable to proteolytic cleavage of carboxy terminal lysine.

2. The DNA of claim 1 which comprises a point mutation in a lysine codon for the mutant creatine kinase subunit carboxy terminal lysine.

3. The DNA of claim 2 wherein the point mutation engenders a stop codon.

4. The DNA molecule of claim 2 having the sequence of Figure 4 or Figure 5 wherein the creatine kinase subunit M is lys⁻M or lys⁻B.

5. A recombinant vector comprising a DNA molecule in accordance with any of claims 1-4.

6. The recombinant vector of claim 5 further defined as an expression vector capable of expressing a creatine kinase subunit in a host cell.

7. The recombinant vector of claim 6 wherein the host cell is a prokaryotic or eukaryotic cell.

8. The recombinant vector of claim 6 wherein the host cell is COS.

9. A method of producing recombinant isoforms of creatine kinase comprising the steps:
transforming or transfecting a host cell with a recombinant vector comprising DNA in accordance with any of claims 1-4 encoding a creatine kinase subunit;
culturing the host cell to produce recombinant creatine kinase isoforms; and
collecting the creatine kinase so produced.

10. The method of claim 9 wherein the host cell is a prokaryotic cell.

11. The method of claim 9 wherein the bacterial host cell is a gram-negative bacterium.

12. The method of claim 9 wherein the host cell is a eukaryotic cell.

13. The method of claim 9 wherein the eukaryotic cell is a COS cell.

14. The method of claim 9 further comprising cotransfecting the host cell with at least one further expression vector encoding a creatine kinase subunit.

15. The method of claim 14 wherein the creatine kinase subunit is mutant or wild-type subunit B, M, sarcomeric or ubiquitous mitochondrial creatine kinase.

16. A host cell comprising a DNA molecule in accordance with any of claims 1-4.

17. The host cell of claim 16 further defined as capable of expressing a mutant creatine kinase subunit having stability toward proteolytic degradation.

18. The host cell of claim 16 further defined as a COS cell.

19. A COS cell transfected with at least two recombinant vectors in accordance with claim 5.

20. The COS cell of claim 19 further comprising transfection with a recombinant molecule encoding a wild-type creatine kinase B or M subunit.

21. A COS cell transfected with at least one recombinant vector in accordance with claim 5 and at least one recombinant vector encoding a creatine kinase B, M, sarcomeric or ubiquitous mitochondrial subunit.

22. A composition useful as an analytical standard, comprising creatine kinase subunit dimers wherein at least one dimer contains a mutant creatine kinase M or B subunit which is stable to proteolytic cleavage of carboxy terminal lysine.

23. The composition of claim 22 wherein the creatine kinase subunits are wild-type or mutant creatine kinase M, creatine kinase B, sarcomeric mitochondrial creatine kinase or ubiquitous mitochondrial creatine kinase.

24. A kit useful for producing isoforms of creatine kinase subunits M and B comprising a first expression vector in accordance with claim 5, the vector being suitably aliquoted into a container.

25. The kit of claim 24 further comprising a second expression vector encoding wild-type creatine kinase subunit M.

26. The kit of claim 25 further comprising a third expression vector encoding wild-type creatine kinase subunit B.

27. The kit of claim 25 further comprising a fourth vector encoding ubiquitous or sarcomeric mitochondrial subunits of creatine kinase.

28. The kit of any of claims 24-27 wherein the vector is aliquoted in an amount suitable for convenient use.

29. A method of assessing damage from a suspected heart attack in an individual comprising collecting a plasma or serum sample from the individual and comparing creatine isoforms with mutant creatine isoform standards consisting of creatine kinase M or B subunits, stable to proteolytic cleavage of carboxy terminal lysine.

30. The method of claim 29 wherein the comparing is by electrophoresis.

31. The method of claim 30 wherein the electrophoresis is nondenaturing.

32. The method of claim 29 wherein the mutant isoform standards are produced by the method of claim 9.

## Patentansprüche

1. DNA-Molekül mit einer codierenden Sequenz für eine mutante M- oder B-Untereinheit der Kreatinkinase, wobei die Untereinheit gegenüber einem proteolytischen Abspalten von Lysin am Carboxylende stabil ist.

2. DNA nach Anspruch 1, die eine Punktmutation in einem Lysin-Codon für das Lysin am Carboxylende der mutanten Untereinheit der Kreatinkinase aufweist.

3. DNA nach Anspruch 2, bei der die Punktmutation ein Stop-Codon hervorbringt.

4. DNA-Moleküle nach Anspruch 2 mit der Sequenz aus Fig. 4 oder Fig. 5, wobei die Untereinheit M der Kreatinkinase lys⁻M oder lys⁻B ist.

5. Rekombinanter Vektor mit einem DNA-Molekül in Übereinstimmung mit einem der Ansprüche 1 bis 4.

6. Rekombinanter Vektor nach Anspruch 5, weiter definiert als ein Expressionsvektor, der in einer Wirtszelle eine Untereinheit der Kreatinkinase exprimieren kann.

7. Rekombinanter Vektor nach Anspruch 6, bei dem die Wirtszelle eine prokaryotische oder eukaryotische Zelle ist.

8. Rekombinanter Vektor nach Anspruch 6, bei dem die Wirtszelle eine COS-Zelle ist.

9. Verfahren zum Erzeugen rekombinanter Isoformen von Kreatinkinase, mit den Schritten:
Transformieren oder Transfizieren einer Wirtszelle mit einem rekombinanten Vektor, der DNA in Übereinstimmung mit einem der Ansprüche 1 bis 4 enthält, die eine Untereinheit der Kreatinkinase codiert;
Kultivieren der Wirtszelle, um rekombinante Isoformen der Kreatinkinase zu erzeugen; und
Gewinnen der so erzeugten Kreatinkinase.

10. Verfahren nach Anspruch 9, bei dem die Wirtszelle eine prokaryotische Zelle ist.

11. Verfahren nach Anspruch 9, bei dem die bakterielle Wirtszelle ein gram-negatives Bakterium ist.

12. Verfahren nach Anspruch 9, bei dem die Wirtszelle eine eukaryotische Zelle ist.

13. Verfahren nach Anspruch 9, bei dem die eukaryotische Zelle eine COS-Zelle ist.

14. Verfahren nach Anspruch 9, das weiter das Cotransfizieren der Wirtszelle mit wenigstens einem weiteren Expressionsvektor umfaßt, der eine Untereinheit der Kreatinkinase codiert.

15. Verfahren nach Anspruch 14, bei dem die Untereinheit der Kreatinkinase eine Mutante, die Wildtyp-Untereinheit B oder M, sarkomische oder allgemein verbreitete mitochondrische Kreatinkinase ist.

16. Wirtszelle mit einem DNA-Molekül in Übereinstimmung mit einem der Ansprüche 1 bis 4.

17. Wirtszelle nach Anspruch 16, ferner dadurch definiert, daß sie eine mutante Untereinheit der Kreatinkinase exprimieren kann, wobei die Untereinheit eine Stabilität gegenüber proteolytischem Abbau aufweist.

18. Wirtszelle nach Anspruch 16, weiter definiert als eine COS-Zelle.

19. COS-Zelle, die mit wenigstens zwei rekombinanten Vektoren in Übereinstimmung mit Anspruch 5 transfiziert ist.

20. COS-Zelle nach Anspruch 19, die weiter eine Transfektion mit einem rekombinanten Molekül aufweist, das eine B- oder M-Untereinheit der Kreatinkinase vom Wildtyp codiert.

21. COS-Zelle, die mit wenigstens einem rekombinanten Vektor in Übereinstimmung mit Anspruch 5 und wenigstens einem rekombinanten Vektor transfiziert ist, der eine B-, M-, sarkomische oder allgemein verbreitete mitochondrische Untereinheit der Kreatinkinase codiert.

22. Zusammensetzung, die als analytischer Standard verwendbar ist, die Dimere von Untereinheiten der Kreatinkinase umfaßt, wobei wenigstens ein Dimer eine mutante M- oder B-Untereinheit der Kreatinkinase umfaßt, die gegenüber einem proteolytischen Abspalten von Lysin am Carboxylende stabil ist.

23. Zusammensetzung nach Anspruch 22, bei der die Untereinheiten der Kreatinkinase Wildtyp- oder mutante Kreatinkinase M, Kreatinkinase B, sarkomische oder mitochondrische Kreatinkinase oder allgemein verbreitete mitochondrische Kreatinkinase sind.

24. Kit, der zur Erzeugung von Isoformen der Untereinheiten M und B der Kreatinkinase nützlich ist, mit einem ersten Expressionsvektor in Übereinstimmung mit Anspruch 5, wobei der Vektor geeignet in einem Behälter aliquotiert ist.

25. Kit nach Anspruch 24, der weiter einen zweiten Expressionsvektor umfaßt, der für die Wildtyp-Untereinheit M der Kreatinkinase codiert.

26. Kit nach Anspruch 25, der weiter einen dritten Expressionsvektor umfaßt, der für die Wildtyp-Untereinheit B der Kreatinkinase codiert.

27. Kit nach Anspruch 25, der weiter einen vierten Vektor umfaßt, der für allgemein verbreitete, sarkomische oder mitochondrische Untereinheiten der Kreatinkinase codiert.

28. Kit nach einem der Ansprüche 24 bis 27, bei dem der Vektor in einer Menge aliquotiert ist, die für zweckdienlichen Einsatz geeignet ist.

29. Verfahren zum Abschätzen von Schäden infolge einer vermuteten Herzattacke in einem Individuum, mit den Schritten:
Gewinnen einer Plasma- oder Serumprobe von dem Individuum und Vergleichen von Kreatin-Isoformen mit mutanten Kreatin-Isoformstandards, die aus M- oder B-Untereinheiten der Kreatinkinase bestehen, wobei die Untereinheiten gegenüber proteolytischem Abspalten von Lysin am Carboxylende stabil sind.

30. Verfahren nach Anspruch 29, bei dem das Vergleichen durch eine Elektrophorese erfolgt.

31. Verfahren nach Anspruch 30, bei dem die Elektrophorese nicht denaturierend ist.

32. Verfahren nach Anspruch 29, bei dem die mutanten Isoformstandards durch das Verfahren nach Anspruch 9 erzeugt werden.

## Revendications

1. Molécule d'ADN comprenant une séquence codante d'une sous-unité de créatine kinase M ou B mutante qui est stable au clivage protéolytique d'une lysine carboxy-terminale.

2. ADN selon la revendication 1, qui comporte une mutation ponctuelle dans un codon de lysine pour la lysine carboxy-terminale de sous-unité de créatine kinase mutante.

3. ADN selon la revendication 2, dans lequel la mutation ponctuelle engendre un codon d'arrêt.

4. Molécule d'ADN selon la revendication 2, possédant la séquence de la figure 4 ou de la figure 5, dans laquelle la sous-unité de créatine kinase M est lys M ou lys B.

5. Vecteur recombinant comprenant une molécule d'ADN conforme à l'une quelconque des revendications 1-4.

6. Vecteur recombinant selon la revendication 5, défini en outre comme étant un vecteur d'expression capable d'exprimer une sous-unité de créatine kinase dans une cellule hôte.

7. Vecteur recombinant selon la revendication 6, dans laquelle la cellule hôte est une cellule procaryote ou eucaryote.

8. Vecteur recombinant selon la revendication 6, dans laquelle la cellule hôte est une cellule COS.

9. Procédé de production d'isoformes recombinantes de créatine kinase comprenant les étapes qui consistent à:
transformer ou transfecter une cellule hôte avec un vecteur recombinant comprortant un ADN conforme à l'une quelconque des revendications 1-4, codant pour une sous-unité de créatine kinase;
cultiver la cellule hôte pour produire des isoformes recombinantes de créatine kinase; et
recueillir la créatine kinase ainsi produite.

10. Procédé selon la revendication 9, dans lequel la cellule hôte est une cellule procaryote.

11. Procédé selon la revendication 9, dans lequel la cellule hôte bactérienne est une bactérie Gram-négatif.

12. Procédé selon la revendication 9, dans lequel la cellule hôte est une cellule eucaryote.

13. Procédé selon la revendication 9, dans lequel la cellule eucaryote est une cellule COS.

14. Procédé selon la revendication 9, comprenant en outre la co-transfection de la cellule hôte avec au moins un autre vecteur d'expression codant pour une sous-unité de créatine kinase.

15. Procédé selon la revendication 14, dans lequel la sous-unité de créatine kinase est une sous-unité B, M mutante ou de type sauvage, une créatine kinase mitochondriale sarcomère ou ubiquiste.

16. Cellule hôte comportant une molécule d'ADN conforme à l'une quelconque des revendications 1-4.

17. Cellule hôte selon la revendication 16, définie en outre comme étant capable d'exprimer une sous-unité de créatine kinase mutante ayant une stabilité vis-à-vis d'une dégradation protéolytique.

18. Cellule hôte selon la revendication 16, définie en outre comme étant une cellule COS.

19. Cellule COS transfectée avec au moins deux vecteurs recombinants conformes à la revendication 5.

20. Cellule COS selon la revendication 19, comprenant en outre la transfection avec une molécule recombinante codant pour une sous-unité de créatine kinase B ou M de type sauvage.

21. Cellule COS transfectée avec au moins un vecteur recombinant conforme à la revendication 5 et au moins un vecteur recombinant codant pour une sous-unité mitochondriale sarcomère ou ubiquiste de créatine kinase B, M.

22. Composition utile comme étalon analytique, comprenant des dimères de sous-unités de créatine kinase dans lesquels au moins un dimère contient une sous-unité de créatine kinase M ou B mutante qui est stable au clivage protéolytique d'une lysine carboxy-terminale.

23. Composition selon la revendication 22, dans laquelle les sous-unités de créatine kinase correspondent à une créatine kinase M, une créatine kinase B, de type sauvage ou mutantes, une créatine kinase mitochondriale sarcomère ou une créatine kinase mitochondriale ubiquiste.

24. Trousse utile pour produire des isoformes de sous-unités de créatine kinase M et B comprenant un premier vecteur d'expression conforme à la revendication 5, le vecteur étant convenablement ajouté par aliquote dans un récipient.

25. Trousse selon la revendication 24, comprenant en outre un second vecteur d'expression codant pour la sous-unité de créatine kinase de type sauvage M.

26. Trousse selon la revendication 25, comprenant en outre un troisième vecteur d'expression codant pour la sous-unité de créatine kinase de type sauvage B.

27. Trousse selon la revendication 25, comprenant en outre un quatrième vecteur codant pour les sous-unités mitochondriales ubiquistes ou sarcomères de créatine kinase.

28. Trousse selon l'une quelconque des revendications 24-27, dans laquelle le vecteur est ajouté par aliquote en une quantité convenable pour une utilisation commode.

29. Procédé d'évaluation des lésions dues à une crise cardiaque présumée chez un individu, comprenant les étapes qui consistent à prélever un échantillon de plasma ou de sérum chez l'individu et à comparer les isoformes de créatine avec des étalons d'isoforme de créatine mutante constitués de sous-unités de créatine kinase M ou B, stables au clivage protéolytique d'une lysine carboxy-terminale.

30. Procédé selon la revendication 29, dans lequel la comparaison s'effectue par électrophorèse.

31. Procédé selon la revendication 30, dans lequel l'électrophorèse est non dénaturante.

32. Procédé selon la revendication 29, dans lequel les étalons d'isoforme mutante sont produits par le procédé selon la revendication 9.
